(19) Europäisches Patentamt
European Patent Office
Office européen des brevets

(11) Veröffentlichungsnummer: **0 409 771 A2**

(12) **EUROPÄISCHE PATENTANMELDUNG**

(21) Anmeldenummer: **90810447.4**

(22) Anmeldetag: **19.06.90**

(51) Int. Cl.5: **D06M 13/355**, C07D 211/94, C08K 5/34

(30) Priorität: **27.06.89 CH 2382/89**

(43) Veröffentlichungstag der Anmeldung: **23.01.91 Patentblatt 91/04**

(84) Benannte Vertragsstaaten: **BE CH DE IT LI NL**

(71) Anmelder: **CIBA-GEIGY AG**
**Klybeckstrasse 141**
**CH-4002 Basel(CH)**

(72) Erfinder: **Reinert, Gerhard, Dr.**
**Weiherweg 1/7**
**CH-4123 Allschwil(CH)**

(54) **Verfahren zur fotochemischen und thermischen Stabilisierung von mit sauren und basischen Farbstoffen anfärbbaren Polyamidfasern und deren Mischungen untereinander und mit anderen Fasern.**

(57) Es wird ein Verfahren zur fotochemischen und thermischen Stabilisierung von mit sauren und basischen Farbstoffen anfärbbaren Polyamidfasern und deren Mischungen untereinander und mit anderen Fasern, ein Mittel zur Ausführung der Verfahrens sowie die damit behandelten Fasermaterialien, beschrieben.

EP 0 409 771 A2

# VERFAHREN ZUR FOTOCHEMISCHEN UND THERMISCHEN STABILISIERUNG VON MIT SAUREN UND BASISCHEN FARBSTOFFEN ANFÄRBBAREN POLYAMIDFASERN UND DEREN MISCHUNGEN UNTEREINANDER UND MIT ANDEREN FASERN

Die vorliegende Erfindung betrifft ein Verfahren zur fotochemischen und thermischen Stabilisierung von mit sauren und basischen Farbstoffen anfärbbaren Polyamidfasern und deren Mischungen untereinander und mit anderen Fasern, ein Mittel zur Ausführung des Verfahrens sowie die damit behandelten Fasermaterialien.

Ungefärbtes und textilveredeltes Polyamidfasermaterial und basisch anfärbbares Polyamid sowie deren Mischungen untereinander und mit anderen Fasern werden ebenso wie die Färbungen dieser Substrate unter Einwirkung von Licht und besonders bei gleichzeitiger Wärmestrahlung geschädigt. So ist die fotochemische Stabilität dieser Fasern, die mit ausgewählten Farbstoffen gefärbt sind, beispielsweise bei der Automobilinnenausstattung, nicht ausreichend.

Aus der EP-A-0 255 481 ist bekannt, die Lichtechtheiten von Polyamidfasergemischen durch Behandlung mit einer Mischung aus Kupferkomplexfarbstoffen, Lichtschutzmitteln und Antioxidantien zu verbessern.

Es wurde nun gefunden, dass durch eine Behandlung dieser Fasern mit Lichtschutzmitteln aus der Klasse der sterisch gehinderten Amine in Abwesenheit von metallhaltigen Verbindungen eine gute Licht- und Wärmestabilisierung erzielt wird. Diese Substanzen können bevorzugt im Färbebad eingesetzt werden.

Diese Lichtschutzmittel finden bevorzugt dort Interesse, wo eine Verwendung metallhaltiger Verbindungen unerwünscht ist, z.B. bei modifizierten PA-Fasern, vor allem aber bei Fasermischungen oder Faserkonstruktionen von Polyamid- mit Polypropylen- oder den verschiedenen Polyurethanfasern.

Gegenstand vorliegender Erfindung ist somit ein Verfahren zur fotochemischen und thermischen Stabilisierung von mit sauren und basischen Farbstoffen anfärbbaren Polyamidfasern und deren Mischungen untereinander und mit anderen Fasern, das dadurch gekennzeichnet ist, dass man das Fasermaterial mit einer wässrigen Flotte, enthaltend ein Lichtschutzmittel aus der Klasse der sterisch gehinderten Amine, behandelt.

Als erfindungsgemäss einzusetzendes Lichtschutzmittel wird ein sterisch gehindertes Amin verwendet, das in seinem Molekül mindestens eine Gruppe der Formel I

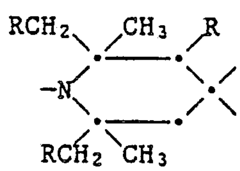

(I)

enthält, worin R Wasserstoff oder Methyl ist.

Solche Lichtschutzmittel können niedermolekular (MG < 700) oder höhermolekular (Oligomere, Polymere) sein. Bevorzugt tragen diese Gruppen einen oder zwei polare Substituenten in 4-Stellung oder ein polares Spiro-Ringsystem ist an die 4-Stellung gebunden.

Von besonderem Interesse sind

a) sterisch gehinderte Amine der Formel II

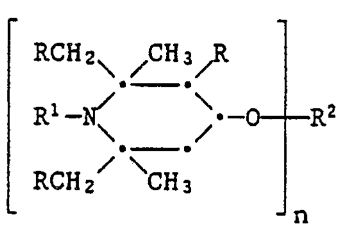

(II),

worin n eine Zahl von 1 bis 4, vorzugsweise 1 oder 2 bedeutet,

R Wasserstoff oder Methyl bedeutet,

$R^1$ Wasserstoff, Hydroxy, $C_1$-$C_{12}$-Alkyl, $C_3$-$C_8$-Alkenyl, $C_3$-$C_8$-Alkinyl, $C_7$-$C_{12}$-Aralkyl, $C_1$-$C_8$-Alkanoyl, $C_3$-$C_5$-Alkenoyl, Glycidyl, -O-$C_1$-$C_{12}$-Alkyl, -O-$C_1$-$C_8$-Alkanoyl oder eine Gruppe -$CH_2CH(OH)$-Z, worin Z Wasserstoff, Methyl oder Phenyl ist, bedeutet, wobei $R^1$ vorzugsweise Wasserstoff, $C_1$-$C_4$-Alkyl, Allyl,

Benzyl, Acetyl oder Acryloyl ist und

$R^2$, wenn n 1 ist, Wasserstoff, gegebenenfalls durch ein oder mehrere Sauerstoffatome unterbrochenes $C_1$-$C_{18}$-Alkyl, Cyanethyl, Benzyl, Glycidyl, einen einwertigen Rest einer aliphatischen, cycloaliphatischen, araliphatischen, ungesättigten oder aromatischen Carbonsäure, Carbaminsäure oder Phosphor enthaltenden Säure oder einen einwertigen Silylrest, vorzugsweise einen Rest einer aliphatischen Carbonsäure mit 2 bis 18 C-Atomen, einer cycloaliphatischen Carbonsäure mit 7 bis 15 C-Atomen, einer $\alpha,\beta$-ungesättigten Carbonsäure mit 3 bis 5 C-Atomen oder einer aromatischen Carbonsäure mit 7 bis 15 C-Atomen bedeutet, wenn n 2 ist, $C_1$-$C_{12}$-Alkylen, $C_4$-$C_{12}$-Alkenylen, Xylylen, einen zweiwertigen Rest einer aliphatischen, cycloaliphatischen, araliphatischen oder aromatischen Dicarbonsäure, Dicarbaminsäure oder Phosphor enthaltenden Säure oder einen zweiwertigen Silylrest, vorzugsweise einen Rest einer aliphatischen Dicarbonsäure mit 2 bis 36 C-Atomen, einer cycloaliphatischen oder aromatischen Dicarbonsäure mit 8 - 14 C-Atomen oder einer aliphatischen, cycloaliphatischen oder aromatischen Dicarbaminsäure mit 8 - 14 C-Atomen bedeutet, wenn n 3 ist, einen dreiwertigen Rest einer aliphatischen, cycloaliphatischen oder aromatischen Tricarbonsäure, einer aromatischen Tricarbaminsäure oder einer Phosphor enthaltenden Säure oder einen dreiwertigen Silylrest bedeutet und wenn n 4 ist, einen vierwertigen Rest einer aliphatischen, cycloaliphatischen oder aromatischen Tetracarbonsäure bedeutet.

Bedeuten etwaige Substituenten $C_1$-$C_{12}$-Alkyl, so stellen sie z.B. Methyl, Ethyl, n-Propyl, n-Butyl, sek.-Butyl, tert.-Butyl, n-Hexyl, n-Octyl, 2-Ethyl-hexyl, n-Nonyl, n-Decyl, n-Undecyl oder n-Dodecyl dar.

In der Bedeutung von $C_1$-$C_{18}$-Alkyl kann $R^1$ oder $R^2$ z.B. die oben angeführten Gruppen und dazu noch beispielsweise n-Tridecyl, n-Tetradecyl, n-Hexadecyl oder n-Octadecyl darstellen.

Wenn $R^1$ $C_3$-$C_8$-Alkenyl bedeutet, so kann es sich z.B. um 1-Propenyl, Allyl, Methallyl, 2-Butenyl, 2-Pentenyl, 2-Hexenyl, 2-Octenyl, 4-tert.-Butyl-2-butenyl handeln.

$R^1$ ist als $C_3$-$C_8$-Alkinyl bevorzugt Propargyl.

Als $C_7$-$C_{12}$-Aralkyl ist $R^1$ insbesondere Phenethyl und vor allem Benzyl.

$R^1$ ist als $C_1$-$C_8$-Alkanoyl beispielsweise Formyl, Propionyl, Butyryl, Octanoyl, aber bevorzugt Acetyl und als $C_3$-$C_5$-Alkenoyl insbesondere Acryloyl.

Bedeutet $R^2$ einen einwertigen Rest einer Carbonsäure, so stellt er beispielsweise einen Essigsäure-, Capronsäure-, Stearinsäure-, Acrylsäure-, Methacrylsäure-, Benzoe- oder $\beta$-(3,5-Di-tert.-butyl-4-hydroxy-phenyl)-propionsäurerest dar.

Bedeutet $R^2$ einen zweiwertigen Rest einer Dicarbonsäure, so stellt er beispielsweise einen Malonsäure-, Bernsteinsäure-, Glutarsäure-, Adipinsäure-, Korksäure-, Sebacinsäure-, Maleinsäure-, Phthalsäure-, Dibutylmalonsäure-, Dibenzylmalonsäure-, Butyl-(3,5-di-tert.-butyl-4-hydroxybenzyl)-malonsäure- oder Bicycloheptendicarbonsäurerest dar.

Stellt $R^2$ einen dreiwertigen Rest einer Tricarbonsäure dar, so bedeutet er z.B. einen Trimellitsäure- oder einen Nitrilotriessigsäurerest.

Stellt $R^2$ einen vierwertigen Rest einer Tetracarbonsäure dar, so bedeutet er z.B. den vierwertigen Rest von Butan-1,2,3,4-tetracarbonsäure oder von Pyromellitsäure.

Bedeutet $R^2$ einen zweiwertigen Rest einer Dicarbaminsäure, so stellt er beispielsweise einen Hexamethylendicarbaminsäure- oder einen 2,4-Toluylen-dicarbaminsäurerest dar.

Beispiele für Polyalkylpiperidin-Verbindungen dieser Klasse sind folgende Verbindungen:

1) 4-Hydroxy-2,2,6,6-tetramethylpiperidin
2) 1-Allyl-4-hydroxy-2,2,6,6-tetramethylpiperidin
3) 1-Benzyl-4-hydroxy-2,2,6,6-tetramethylpiperidin
4) 1-(4-tert.-Butyl-2-butenyl)-4-hydroxy-2,2,6,6-tetramethylpiperidin
5) 4-Stearoyloxy-2,2,6,6-tetramethylpiperidin
6) 1-Ethyl-4-salicyloyloxy-2,2,6,6-tetramethylpiperidin
7) 4-Methacryloyloxy-1,2,2,6,6-pentamethylpiperidin
8) 1,2,2,6,6-Pentamethylpiperidin-4-yl-$\beta$-(3,5-di-tert.-butyl-4-hydroxyphenyl)-propionat
9) Di-(1-benzyl-2,2,6,6-tetramethylpiperidin-4-yl)-maleinat
10) Di-(2,2,6,6-tetramethylpiperidin-4-yl)-succinat
11) Di-(2,2,6,6-tetramethylpiperidin-4-yl)-glutarat
12) Di-(2,2,6,6-tetramethylpiperidin-4-yl)-adipat
13) Di-(2,2,6,6-tetramethylpiperidin-4-yl)-sebacat
14) Di-(1,2,2,6,6-pentamethylpiperidin-4-yl)-sebacat
15) Di-(1,2,3,6-tetramethyl-2,6-diethyl-piperidin-4-yl)-sebacat
16) Di-(1-allyl-2,2,6,6-tetramethylpiperidin-4-yl)-phthalat
17) 1-Propargyl-4-$\beta$-cyanoethyloxy-2,2,6,6-tetramethylpiperidin
18) 1-Acetyl-2,2,6,6-tetramethylpiperidin-4-yl-acetat

19) Trimellithsäure-tri-(2,2,6,6-tetramethylpiperidin-4-yl)-ester
20) 1-Acryloyl-4-benzyloxy-2,2,6,6-tetramethylpiperidin
21) Diethylmalonsäure-di(2,2,6,6-tetramethylpiperidin-4-yl)-ester
22) Dibutyl-malonsäure-di-(1,2,2,6,6-pentamethylpiperidin-4-yl)-ester
23) Butyl-(3,5-di-tert.-butyl-4-hydroxybenzyl)-malonsäure-di-(1,2,2,6,6-pentamethylpiperidin-4-yl)-ester
24) Dibenzyl-malonsäure-di-(1,2,2,6,6-pentamethylpiperidin-4-yl)-ester
25) Dibenzyl-malonsäure-di-(1,2,3,6-tetramethyl-2,6-diethyl-piperidin4-yl)ester
26) Hexan-1',6'-bis-(4-carbamoyloxy-1-n-butyl-2,2,6,6-tetramethylpiperidin)
27) Toluol-2',4'-bis-(4-carbamoyloxy-1-n-propyl-2,2,6,6-tetramethylpiperidin)
28) Dimethyl-bis-(2,2,6,6-tetramethylpiperidin-4-oxy)-silan
29) Phenyl-tris-(2,2,6,6-tetramethylpiperidin-4-oxy)-silan
30) Tris-(1-propyl-2,2,6,6-tetramethylpiperidin-4-yl)-phosphit
31) Tris-(1-propyl-2,2,6,6-tetramethylpiperidin-4-yl)phosphat
32) Phenyl-[bis-(1,2,2,6,6-pentamethylpiperidin-4-yl)]-phosphonat
33) 4-Hydroxy-1,2,2,6,6-pentamethylpiperidin
34) 4-Hydroxy-N-hydroxyethyl-2,2,6,6-tetramethylpiperidin
35) 4-Hydroxy-N-(2-hydroxypropyl)-2,2,6,6-tetramethylpiperidin
36) 1-Glycidyl-4-hydroxy-2,2,6,6-tetramethylpiperidin

b) Verbindungen der Formel (III)

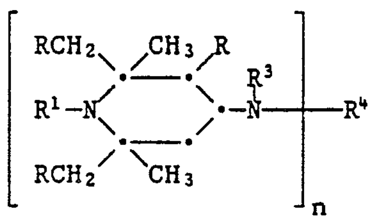

(III)

worin n die Zahl 1 oder 2 bedeutet,
R und $R^1$ die unter a) angegebene Bedeutung haben,
$R^3$ Wasserstoff, $C_1$-$C_{12}$-Alkyl, $C_2$-$C_5$-Hydroxyalkyl, $C_5$-$C_7$-Cycloalkyl, $C_7$-$C_8$-Aralkyl, $C_2$-$C_{18}$-Alkanoyl, $C_3$-$C_5$-Alkenoyl oder Benzoyl ist und
$R^4$, wenn n 1 ist, Wasserstoff, $C_1$-$C_{18}$-Alkyl, $C_3$-$C_8$-Alkenyl, $C_5$-$C_7$-Cycloalkyl, mit einer Hydroxy-, Cyano-, Alkoxycarbonyl- oder Carbamidgruppe substituiertes $C_1$-$C_4$-Alkyl, Glycidyl, eine Gruppe der Formel $-CH_2-CH(OH)-Z$ oder der Formel -CONH-Z ist, worin Z Wasserstoff, Methyl oder Phenyl bedeutet; wenn n 2 ist, $C_2$-$C_{12}$-Alkylen, $C_6$-$C_{12}$-Arylen, Xylylen, eine $-CH_2-CH(OH)-CH_2$-Gruppe oder eine Gruppe $-CH_2-CH(OH)-CH_2-O-O-O-$ bedeutet, worin D $C_2$-$C_{10}$-Alkylen, $C_6$-$C_{15}$-Arylen, $C_6$-$C_{12}$-Cycloalkylen ist, oder vorausgesetzt, dass $R^3$ nicht Alkanoyl, Alkenoyl oder Benzoyl bedeutet, $R^4$ auch einen zweiwertigen Rest einer aliphatischen, cycloaliphatischen oder aromatischen Dicarbonsäure oder Dicarbaminsäure oder auch die Gruppe -CO- bedeuten kann, oder $R^3$ und $R^4$ zusammen, wenn n 1 ist, den zweiwertigen Rest einer aliphatischen, cycloaliphatischen oder aromatischen 1,2- oder 1,3-Dicarbonsäure bedeuten können.

Stellen etwaige Substituenten $C_1$-$C_{12}$- oder $C_1$-$C_{18}$-Alkyl dar, so haben sie die unter a) angegebene Bedeutung.

Bedeuten etwaige Substituenten $C_5$-$C_7$-Cycloalkyl, so stellen sie insbesondere Cyclohexyl dar.

Als $C_7$-$C_8$-Aralkyl ist $R^3$ insbesondere Phenylethyl oder vor allem Benzyl. Als $C_2$-$C_5$-Hydroxyalkyl ist $R^3$ insbesondere 2-Hydroxyethyl oder 2-Hydroxypropyl.

$R^3$ ist als $C_2$-$C_{18}$-Alkanoyl beispielsweise Propionyl, Butyryl, Octanoyl, Dodecanoyl, Hexadecanoyl, Octadecanoyl, aber bevorzugt Acetyl und als $C_3$-$C_5$-Alkenoyl insbesondere Acryloyl.

Bedeutet $R^4$ $C_2$-$C_8$-Alkenyl, dann handelt es sich z.B. um Allyl, Methallyl, 2-Butenyl, 2-Pentenyl, 2-Hexenyl oder 2-Octenyl.

$R^4$ als mit einer Hydroxy-, Cyano-, Alkoxycarbonyl- oder Carbamidgruppe substituiertes $C_1$-$C_4$-Alkyl kann z.B. 2-Hydroxyethyl, 2-Hydroxypropyl, 2-Cyanethyl, Methoxycarbonylmethyl, 2-Ethoxycarbonylethyl, 2-Aminocarbonylpropyl oder 2-(Dimethylaminocarbonyl)-ethyl sein.

Stellen etwaige Substituenten $C_2$-$C_{12}$-Alkylen dar, so handelt es sich z.B. um Ethylen, Propylen, 2,2-Dimethylpropylen, Tetramethylen, Hexamethylen, Octamethylen, Decamethylen oder Dodecamethylen.

Bedeuten etwaige Substituenten $C_6$-$C_{15}$-Arylen, so stellen sie z.B. o-, m- oder p-Phenylen, 1,4-Naphthylen oder 4,4'-Diphenylen dar.

Als $C_6$-$C_{12}$-Cycloalkylen ist D insbesondere Cyclohexylen.

Beispiele für Polyalkylpiperidin-Verbindungen dieser Klasse sind folgende Verbindungen:

37) N,N′-Bis-(2,2,6,6-tetramethylpiperidin-4-yl)-hexamethylen-1,6-diamin

38) N,N′-Bis-(2,2,6,6-tetramethylpiperidin-4yl)-hexamethylen-1,6-diacetamid

39) 1-Acetyl-4-(N-cyclohexylacetamido)-2,2,6,6-tetramethylpiperidin

40) 4-Benzoylamino-2,2,6,6-tetramethylpiperidin

41) N,N′-Bis-(2,2,6,6-tetramethylpiperidin-4-yl)-N,N′-dibutyl-adipamid

42) N,N′-Bis-(2,2,6,6-tetramethylpiperidin-4-yl)-N,N′-dicyclohexyl-2-hydroxypropylen-1,3-diamin

43) N,N′-Bis-(2,2,6,6-tetramethylpiperidin4-yl)-p-xylylen-diamin

44) N,N′-Bis-(2,2,6,6-tetramethylpiperidin-4-yl)-succindiamid

45) N-(2,2,6,6-Tetramethylpiperidin-4-yl)-$\beta$-aminodipropionsäure-di-(2,2,6,6-tetramethylpiperidin-4-yl)-ester

46) Die Verbindung der Formel

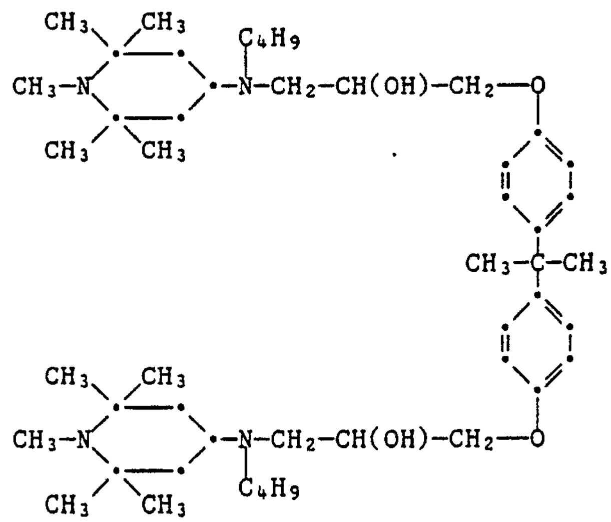

47) 4-(Bis-2-hydroxyethyl-amino)-1,2,2,6,6-pentamethylpiperidin

48) 4-(3-Methyl-4-hydroxy-5-tert.-butyl-benzoesäureamido)-2,2,6,6-tetramethylpiperidin

49) 4-methacrylamido-1,2,2,6,6-pentamethylpiperidin

c) Verbindungen der Formel IV

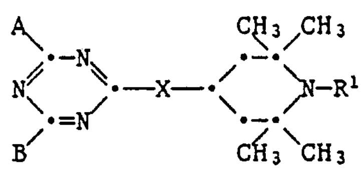 (IV)

worin $R^1$ Wasserstoff, Oxyl-Sauerstoff, $C_1$-$C_{12}$-Alkyl, $C_3$-$C_7$-Alkenyl, $C_7$-$C_{11}$-Phenylalkyl, Cyanomethyl, $C_2$-$C_{18}$-Alkanoyl oder $C_3$-$C_{18}$-Alkenoyl, eine Gruppe -CON($R^2$)($R^3$) oder eine Gruppe -$CH_2$-CH($R^4$)-OH bedeutet, worin

$R^2$ $C_1$-$C_{12}$-Alkyl, Allyl, Cyclohexyl, Benzyl, Phenyl oder $C_7$-$C_{12}$-Alkylphenyl und

$R^3$ Wasserstoff, $C_1$-$C_{12}$-Alkyl, Allyl oder Benzyl bedeuten oder

$R^2$ und $R^3$ zusammen mit dem H-Atom, an das sie gebunden sind, einen 5-oder 6-gliedrigen heterocyclischen Ring bilden, und

$R^4$ Wasserstoff, $C_1$-$C_{12}$-Alkyl, Phenyl, $C_2$-$C_{13}$-Alkoxymethyl oder Phenoxymethyl bedeutet,

X eine zweiwertige Gruppe der Formel -O-, -N($R^5$)-, -NH-($CH_2$)$_2$-O-, -NH-($CH_2$)$_3$-O- oder -N($R^5$)-$R^7$-H($R^6$)-bedeutet, worin

$R^5$ Wasserstoff, $C_1$-$C_{12}$-Alkyl, $C_3$-$C_7$-Alkenyl, Cyclohexyl, $C_3$-$C_{12}$-Alkoxyalkyl, $C_5$-$C_{12}$-Alkenoxyalkyl, $C_4$-$C_{12}$-Dialkylaminoalkyl, eine Gruppe -$CH_2$-CH($R^4$)-OH, Benzyl, eine Gruppe der Formel

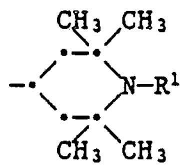

oder der Formel

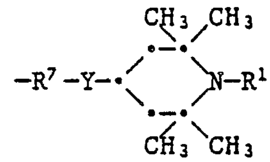

bedeutet,
$R^6$ Wasserstoff, $C_1$-$C_{12}$-Alkyl, $C_3$-$C_7$-Alkenyl, Cyclohexyl, eine Gruppe -$CH_2$-$CH(R^4)$-OH oder eine Gruppe der Formel

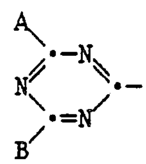

bedeutet,
$R^7$ $C_2$-$C_{12}$-Alkylen, das durch 1, 2 oder 3 der Gruppen -O- oder -$N(R^6)$-unterbrochen sein kann, $C_6$-$C_{14}$-Cycloalkylen oder Cycloalkylendialkylen bedeutet,
Y eine zweiwertige Gruppe der Formel -O- oder -$N(R^6)$- bedeutet und A und B, unabhängig voneinander,
(a) eine Gruppe der Formel $R^8O$- oder $(R^9)(R^{10})$ N- bedeuten, worin $R^8$ $C_1$-$C_{12}$-Alkyl, $C_3$-$C_7$-Alkenyl, Cyclohexyl, Benzyl, Phenyl oder $C_7$-$C_{12}$-Alkylphenyl bedeutet,
$R^9$ $C_1$-$C_{12}$-Alkyl, $C_3$-$C_7$-Alkenyl, $C_5$-$C_8$-Cycloalkyl, $C_3$-$C_{12}$-Alkoxyalkyl, $C_5$-$C_{12}$-Alkenoxyalkyl, eine Gruppe -$CH_2$-$CH(R^4)$-OH, Phenyl, $C_7$-$C_{12}$-Alkylphenyl oder $C_7$-$C_{11}$-Phenylalkyl bedeutet und
$R^{10}$ Wasserstoff, $C_1$-$C_{12}$-Alkyl, $C_3$-$C_7$-Alkenyl, $C_5$-$C_8$-Cycloalkyl, $C_3$-$C_{12}$-Alkoxyalkyl, $C_5$-$C_{12}$-Alkenoxyalkyl, eine Gruppe -$CH_2$-$CH(R^4)$-OH oder $C_7$-$C_{11}$-Phenylalkyl bedeutet, oder $R^9$ und $R^{10}$ zusammen mit dem N-Atom, an das sie gebunden sind, einen 5- oder 6-gliedrigen heterocyclischen Ring bilden.

Die Substituenten $R^1$, $R^2$, $R^4$, $R^5$, $R^6$, $R^8$, $R^9$ und $R^{10}$ als Alkyl können unverzweigtes oder verzweigtes Alkyl sein, vorzugsweise unverzweigtes. Beispiele für solche Alkylgruppen sind Methyl, Ethyl, Propyl, Butyl, sec.Butyl, n-Hexyl, n-Octyl, 2-Ethylhexyl, n-Decyl oder n-Dodecyl.

$R^1$, $R^5$, $R^6$, $R^8$ und $R^9$ als Alkenyl können unverzweigtes oder verzweigtes Alkenyl bedeuten, wie z.B. Allyl, Methallyl, 2-Butenyl-1, 2-Methyl-2-butenyl-1 oder 2-Hexenyl-1, wobei Allyl bevorzugt ist.

$R^9$ als Cycloalkyl kann z.B. Cyclopentyl, Cyclohexyl, 4-Methylcyclohexyl, Cycloheptyl oder Cyclooctyl sein, wobei Cyclohexyl bevorzugt ist.

$R^1$, $R^9$ und $R^{10}$ als Phenylalkyl können z.B. Butyl, 1-Phenylethyl, 2-Phenylethyl, 3-Phenylpropyl oder 3-Phenylbutyl sein, wobei Benzyl bevorzugt ist.

$R^2$, $R^8$ und $R^9$ als Alkylphenyl können z.B. 4-Tolyl, 2-Tolyl, 3,5-Dimethylphenyl, 4-Ethylphenyl oder 4-Isopropylphenyl sein, wobei 4-Tolyl bevorzugt ist.

$R^1$ als Alkanoyl kann z.B. Acetyl, Propionyl, Butyryl, Hexanoyl (Capronyl), 2-Ethylhexanoyl, n-Octanoyl (Capryloyl), n-Decanoyl (Caprinoyl), n-Dodecanoyl (Lauroyl), n-Hexadecanoyl (Palmitoyl) oder n-Octadecanoyl (Stearoyl) sein. $R^1$ als Alkenoyl kann z.B. Acryloyl, Methacryloyl, Crotonyl, Vinylacetyl oder Oleyl sein.

$R^4$ als Alkoxymethyl kann z.B. Methoxy-, Ethoxy-, Butoxy-, Hexyloxy-, Octyloxy- oder Dodecycloxymethyl sein.

$R^5$ und $R^9$ als Alkoxyalkyl können z.B. 2-Methoxyethyl, 2-Ethoxyethyl, 2-Isopropoxyethyl, 2-Butoxyethyl, 2-Methoxypropyl, 4-Methoxybutyl, 3-Butoxypropyl oder 2-Octyloxyethyl sein. $R^5$ und $R^9$ als Alkenoxyalkyl können z.B. 2-Allyloxyethyl, 2-Methallyloxypropyl oder 3-Allyloxypropyl sein.

$R^5$ als Dialkylaminoalkyl kann insbesondere Dialkylaminopropyl sein, wie z.B. 3-Dimethylamino-, 3-Diethylamino- oder 3-Diisopropylaminopropyl.

$R^7$ als Alkylen oder durch -O- oder -N($R^6$)- unterbrochenes Alkylen kann z.B. 1,2-Ethylen, 1,3-Propylen, 1,4-Butylen, 1,6-Hexylen, 1,8-Octylen, 2,4-Dimethyl-1,6-hexylen, 1,12-Dodecylen, 4-Oxaheptylen-1,7,4-(Methylaza)-heptylen-1,7 oder 4,8-Diazaundecylen-1,11 sein.

$R^7$ als Cycloalkylen oder Cycloalkylen-dialkylen kann z.B. 1,4-Cyclohexylen, 1,5-Cyclooctylen, 1,4-Dimethylencyclohexan oder 3,3-Dimethyl-5-methylencyclohexyl sein.

$R^2$ und $R^3$ sowie $R^9$ und $R^{10}$ können zusammen mit dem N-Atom, an das sie gebunden sind, einen heterocyclischen Ring bilden, wie z.B. einen Pyrrolidin-, Piperidin-, Morpholin- oder 4-Methylpiperazinring.

Unter den Verbindungen der Formel IV sind bevorzugt:

1a) Verbindungen der Formel IV, worin mindestens zwei der Gruppen $R^1$, $R^2$, $R^3$, $R^5$, $R^6$, $R^8$, $R^9$ oder $R^{10}$ Alkenyl oder Alkenoyl sind.

1b) Verbindungen der Formel IV, worin der Substituent A eine Gruppe $R^{15}$O-oder ($R^{15}$)($R^{10}$)N- bedeutet und $R^{15}$ $C_3$-$C_7$-Alkenyl ist.

1c) Verbindungen der Formel IV, worin A und B eine Gruppe der Formel $R^{15}$ O-oder ($R^{15}$)($R^{10}$)N-bedeutet und $R^{15}$ Allyl ist.

1d) Verbindungen der Formel IV, worin die Substituenten A und B eine Gruppe $R^8$O-oder ($R^9$)($R^{10}$)N-bedeuten, worin $R^8$ und $R^9$ Allyl sind, und X eine Gruppe -N($R^5$)- ist, worin $R^5$ eine Gruppe der Formel

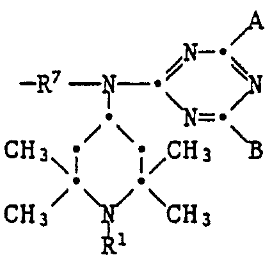

bedeutet und A, B, $R^1$ und $R^7$ die oben angegebene Bedeutung haben.

Beispiele für Verbindungen der Formel (IV) sind:

2,4-Bis[N-(2,2,6,6-tetramethylpiperidyl-4)-butylamino]-6-allylamino-1,3,5-triazin, Fp. 101-103 °C.

N,N'-Bis[2,4-bis(diallylamino)-1,3,5-triazinyl-6]-N,N'-bis(2,2,6,6-tetramethylpiperidyl-4)-hexylmethylendiamin, Fp. 124-125 °C.

2,4-Bis(diallylamino)-6-[N-(2,2,6,6-tetramethylpiperidyl-4)-butylamino]-1,3,5-triazin, viskoses Oel Kp 230 °C/0,1 Pa,

N,N'-Bis[2,4-di(allyloxy)-1,3,5-triazinyl-6]-N,N'-bis(2,2,6,6-tetramethylpiperidyl-4)-hexylmethylendiamin, Fp. 112-113 °C und

N,N'-Bis[2,4-di(allylamino)-1,3,5-triazinyl-6]-N,N'-bis(2,2,6,6-tetramethylpiperidyl-4)-hexylmethylendiamin, Fp. 162-163 °C.

Von ganz besonderem Interesse sind diejenigen Verbindungen der Formel (IV), bei denen A und B eine Gruppe der Formel $R^{15}$O- oder ($R^{15}$)($R^{10}$)N- bedeuten und $R^{15}$ Allyl ist.

Die Verbindungen der Formel (II) und (III) sind bekannt, z.B. aus der US-A-3 840 494, 3 640 928 und 3 993 655 und können nach den dort beschriebenen Verfahren hergestellt werden.

Die Herstellung der Verbindungen der Formel (IV) kann in Analogie zum Verfahren der US-A-3 925 376 erfolgen. Danach wird Cyanurchlorid stufenweise mit den Komponenten AH, BH und einem 4-Hydroxy- oder 4-Amino-2,2,6,6-tetramethylpiperidin umgesetzt. Die Einführung des Substituenten $R^1$ an den Piperidin-Stickstoff kann vor oder nach der Umsetzung mit dem Halogentriazin erfolgen.

Die Verbindungen der Formeln (I) bis (IV) werden zweckmässig als Emulsionen oder fein verteilte Dispersionen eingesetzt, die durch Mahlen oder in Gegenwart von nichtionogenen oder anionischen Dispergiermitteln erhalten werden.

Als nichtionogene Dispergatoren kommen Alkohol- oder Alkylphenolalkylenoxidumsetzungsprodukte, z.B. Alkylenoxidumsetzungsprodukte von aliphatischen Alkoholen mit 4 bis 22 Kohlenstoffatomen, die bis zu 80 Mol Ethylenoxid und/oder Propylenoxid angelagert enthalten, in Betracht. Die Alkohole können vorzugsweise 4 bis 18 Kohlenstoffatome enthalten, sie können gesättigt, verzweigt oder geradkettig sein und können allein oder im Gemisch eingesetzt werden. Alkohole mit verzweigten Ketten sind bevorzugt.

Es können natürliche Alkohole, wie z.B. Myristylalkohol, Cetylalkohol, Stearylalkohol, Oleylalkohol, Arachidylalkohol oder Behenylalkohol oder synthetische Alkohole, wie insbesondere Butanol, 2-Ethylhexanol, Amylalkohol, n-Hexanol, ferner Triethylhexanol, Trimethylnonylalkohol oder Gemische aus höheren Fettalkoholen verwendet werden. Es handelt sich dabei um lineare primäre Alkohole.

Bevorzugte Ethylenoxid-Alkohol-Umsetzungsprodukte können z.B. durch die Formel
$R_3O(CH_2CH_2O)_sH$
dargestellt werden, worin $R^3$ ein gesättigter oder ungesättigter Kohlenwasserstoffrest, vorzugsweise ein Alkyl- oder Alkenylrest mit 8 bis 18 Kohlenstoffatomen und s eine ganze Zahl von 1 bis 80, vorzugsweise von 1 bis 30 ist.

Als nichtionogene Dispergatoren kommen ferner Umsetzungsprodukte aus Ethylenoxid und/oder 1,2-Propylenoxid und Alkylphenolen mit 4 bis 12 Kohlenstoffatomen im Alkylteil, wobei das Phenol ein oder mehrere Alkylsubstituenten enthalten kann, in Betracht. Vorzugsweise entsprechen diese Verbindungen der Formel

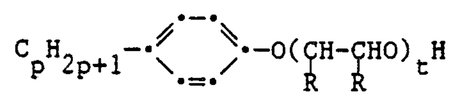

worin R Wasserstoff oder höchstens einer der beiden Reste R Methyl, p eine Zahl von 4 bis 12, vorzugsweise 8 bis 9, und t eine Zahl von 1 bis 60, insbesondere von 1 bis 20 und vorzugsweise 1 bis 6 ist.

Gegebenenfalls können diese Alkohol-Alkylphenol-Ethylenoxid/1,2-Pro pylenoxid-Addukte noch kleinere Anteile von Blockpolymeren aus den genannten Alkylenoxiden enthalten.

Weitere Umsetzungsprodukte, die als nichtionogene Dispergatoren in Betracht kommen, sind Polyoxyethylenderivate der Fettsäureester der Ether des Sorbitans mit 4 Mol Polyethylenglykol, z.B. das Laurat, Palmitat, Stearat, Tristearat, Oleat und Trioleat der genannten Ether wie die Tween-Marken der Atlas Chemicals Division. Bevorzugt ist das Tristearat des Ethers des Sorbitans mit 4 Mol des Polyethylenglykols der Formel
$H(CH_2CH_2)_{65}OH$.

Als anionische Dispergatoren kommen veresterte Alkylenoxidaddukte, wie z.B. saure Estergruppen von anorganischen oder organischen Säuren enthaltende Anlagerungsprodukte von Alkylenoxiden, besonders Ethylenoxid und/oder Propylenoxid, an aliphatische, insgesamt mindestens 8 Kohlenstoffatome aufweisende organische Hydroxyl-, Carboxyl-, gegebenenfalls auch Amino- oder Amidoverbindungen bzw. Mischungen dieser Verbindungen in Betracht. Diese sauren Ester können als freie Säuren oder als Salze, z.B. Alkalimetall-, Erdalkalimetall-, Ammonium- oder Aminsalze vorliegen.

Die Herstellung dieser anionischen Dispergatoren erfolgt nach bekannten Methoden, indem man an die genannten organischen Verbindungen mindestens 1 Mol, vorzugsweise mehr als 1 Mol, z.B. 2 bis 60 Mol Ethylenoxid oder alternierend in beliebiger Reihenfolge Ethylenoxid und Propylenoxid anlagert und anschliessend die Anlagerungsprodukte verethert bzw. verestert und gegebenenfalls die Ether bzw. die Ester in ihre Salze überführt. Als Ausgangsstoffe kommen z.B. höhere Fettalkohole, d.h. Alkanole oder Alkenole mit 8 bis 22 Kohlenstoffatomen, alicyclische Alkohole, Phenylphenole, Alkylphenole mit einem oder mehreren Alkylsubstituenten, der bzw. die zusammen mindestens 10 Kohlenstoffatome aufweisen oder Fettsäuren mit 8 bis 22 Kohlenstoffatomen in Betracht.

Besonders geeignete anionische Dispergatoren entsprechen der Formel

$$(V) \qquad R_1-A-(CH_2\underset{R_2}{CH}O)_n-Q \; ,$$

worin $R^1$ ein aliphatischer Kohlenwasserstoffrest mit 8 bis 22 Kohlenstoffatomen oder ein cycloaliphatischer, aromatischer oder aliphatischaromatischer Kohlenwasserstoffrest mit 10 bis 22 Kohlenstoffatomen, $R^2$ Wasserstoff oder Methyl, A -O- oder

$$-\underset{\overset{\|}{O}}{C}-O,$$

Q der Säurerest einer anorganischen, Sauerstoff enthaltenden Säure, der Säurerest einer mehrbasischen Carbonsäure oder ein Carboxylalkylrest und n eine Zahl von 1 bis 50 ist.

Der Rest $R^1$-A- in den Verbindungen der Formel (V) leitet sich z.B. von höheren Alkoholen wie Decyl-, Lauryl-, Tridecyl-, Myristyl-, Cetyl-, Stearyl-, Oleyl-, Arachidyl- oder Behenylalkohol ab; ferner von alicyclischen Alkoholen, wie Hydroabietylalkohol; von Fettsäuren, wie Capryl-, Caprin-, Laurin-, Myristin-, Palmitin-,

Stearin-, Arachin-, Behen-, Kokosfett- ($C_8$-$C_{18}$), Decen-, Dodecen-, Tetradecen-, Hexadecen-, Oel-, Linol-, Linolen-, Eikosen-, Dokosen- oder Clupanodonsäure; von Alkylphenolen, wie Butyl-, Hexyl-, n-Octyl-, n-Nonyl-, p-tert. Octyl-, p-tert. Nonyl-, Decyl-, Dodecyl-, Tetradecyl- oder Hexadecylphenol oder von Arylphenolen, wie den o- oder p-Phenylphenolen. Bevorzugt sind Reste mit 10 bis 18 Kohlenstoffatomen, insbesondere solche, die sich von den Alkylphenolen ableiten.

Der Säurerest Q ist in der Regel der Säurerest einer mehrbasischen, insbesondere niedermolekularen Mono- oder Dicarbonsäure, wie z.B. von Maleinsäure, Malonsäure, Bernsteinsäure oder Sulfobernsteinsäure, oder ist ein Carboxyalkylrest, insbesondere ein Carboxymethylrest (abgeleitet insbesondere von Chloressigsäure) und ist über eine Ether- oder Esterbrücke mit dem Rest $R_1$-A-($CH_2CHR_1O)_m$- verbunden. Insbesondere leitet sich Q jedoch von anorganischen mehrbasischen Säuren ab, wie Orthophosphorsäure und Schwefelsäure. Der Säurerest X liegt vorzugsweise in Salzform, d.h. z.B. als Alkalimetall-, Ammonium- oder Aminsalz, vor.

Beispiele für solche Salze sind Natrium-, Kalium-, Ammonium-, Trimethylamin-, Ethanolamin-, Diethanolamin- oder Triethanolaminsalze. Bei den Alkylenoxideinheiten $\{CH_2CHR^2O\}$ der Formel (v) handelt es sich in der Regel um Ethylenoxid und 1,2-Propylenoxideinheiten, letztere befinden sich vorzugsweise im Gemisch mit Ethylenoxideinheiten in den Verbindungen der Formel (V).

Von besonderem Interesse sind die anionischen Verbindungen der Formel

$R_3O(CH_2CH_2O)_n$-X ,

worin $R_3$ ein gesättigter oder ungesättigter Kohlenwasserstoffrest mit 8 bis 22 Kohlenwasserstoffatomen, o-Phenylphenol oder Alkylphenyl mit 4 bis 12 Kohlenstoffatomen im Alkylteil ist, und X und n die angegebenen Bedeutungen haben.

Von den Verbindungen, die sich von Alkylphenyl-Ethylenoxidaddukten ableiten, sind ferner solche der Formeln

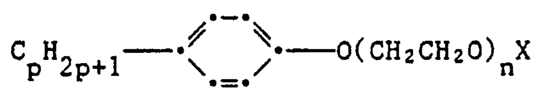

und

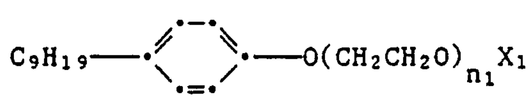,

worin p eine Zahl von 4 bis 12, n eine ganze Zahl von 1 bis 20, $n_1$ eine ganze Zahl von 1 bis 10, $X_1$ ein Schwefelsäure oder Phosphorsäurerest, der gegebenenfalls in Salzform vorliegt und X die angegebene Bedeutung hat, besonders bevorzugt.

Als weitere Dispergatoren kommen z.B. die bekannten Ligninsulfonate, Kondensationsprodukte von Naphthalinsulfonsäure und/oder Naphthol- oder Naphthylaminsulfonsäuren mit Formaldehyd, Kondensationsprodukte von Phenolsulfonsäuren und/oder Phenolen mit Formaldehyd und Harstoff in Betracht.

Als Dispergiermittel besonders geeignet sind auch sulfonierte Kondensationsprodukte, welche durch Umsetzung einer mindestens zwei ersetzbare kernständige Wasserstoffatome aufweisenden aromatischen Verbindung in beliebiger Reihenfolge mit einer Verbindung der Formel

(VI) 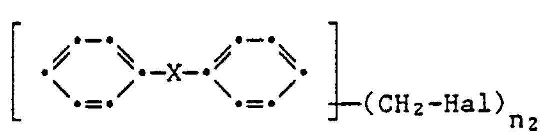,

worin X die direkte Bindung oder Sauerstoff,
Hal Chlor oder Brom und
$n_2$ 1 bis 4 bedeuten
und Sulfonierung erhalten worden sind.

Diese sulfonierten Kondensationsprodukte entsprechen vorzugsweise der Formel

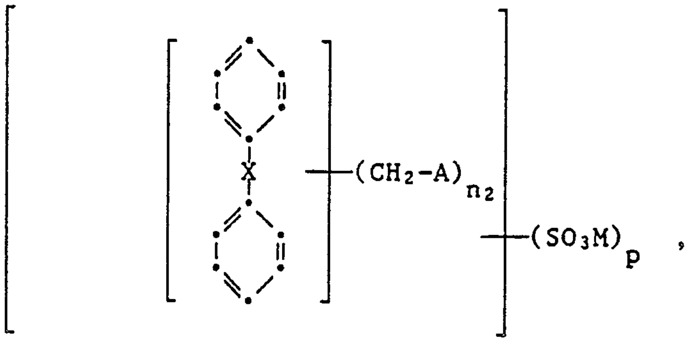

in der X die direkte Bindung oder Sauerstoff, A den Rest einer aromatischen Verbindung, welcher mittels eines Ringkohlenstoffatoms an die Methylengruppe gebunden ist, M Wasserstoff oder ein Kation, z.B. Alkalimetall, Erdalkalimetall, Ammoniumgruppe und n und p je eine Zahl von 1 bis 4 bedeuten. Dabei stehen $n_2$ und p vorzugsweise für 1 oder 2, oder sie können auch je eine beliebige gebrochene Zahl von 1 bis 4 bedeuten z.B. 1,4, 1,8, 2,1 oder 3,2.

Aromatischen Verbindungen, die mindestens zwei ersetzbare Wasserstoffatome aufweisen, können ein- oder mehrkernige, besonders zweikernige Kohlenwasserstoffe sein, die gegebenenfalls substituiert sind. Als Substituenten kommen z.B. Hydroxy-, Alkylgruppen mit 1 bis 4 Kohlenstoffatomen, Alkoxygruppen mit 1 bis 4 Kohlenstoffatomen oder Halogen, wie z.B. Chlor in Betracht. Bevorzugt sind Naphthalinverbindungen, die mit Hydroxyl, Chlor oder Methyl substituiert sein können. Als Beispiele für ein- und mehrkernige aromatische Verbindungen seien hier genannt:

Alkylbenzole, wie z.B. Toluol, Xylole, Isopropylbenzol, Isobutylbenzol, tert.Butylbenzol; Phenol, Chlorphenole, Alkylphenole, wie z.B. Methylphenol, Dimethylphenol, Isopropylphenol oder tert.Butylphenol, Hydroxydiphenyle; Alkoxybenzole, wie z.B. Anisole, Phenetole, Butoxybenzol, Diphenylalkane, Hydroxydiphenylalkane, Tetrahydronaphthalin, Naphthalin, α- und β-Naphthol, Alkylnaphthaline, z.B. α- und β-Methylnaphthalin, sowie Acenaphthen, Anthracen, Perylen, Pyren, Dihydrophenanthren oder Phenanthren. Besonders gut geeignet ist Naphthalin, das auch bereits sulfoniert sein kann. Selbstverständlich können auch Gemische dieser ein- und mehrkernigen aromatischen Verbindungen als Ausgangsstoffe verwendet werden.

Die ebenfalls als Ausgangsstoffe benötigten Verbindungen der Formel (VI) werden z.B. durch Umsetzung von Diphenyl oder Diphenylether mit Formaldehyd und Halogenwasserstoff, wie Brom- oder vorzugsweise Chlorwasserstoff nach den in der U.S. Patentschrift 3 004 072 oder italienischen Patentschrift 600 214 beschriebenen Methoden hergestellt.

Bevorzugte Ausgangsstoffe der Formel (VI) sind Chlormethyldiphenyl und Chlormethyldiphenylether. Bei diesen Verbindungen handelt es sich meistens um Isomerengemische mit 1 bis 3 Chlormethylgruppen, wobei sich z.B. die Chlormethylgruppen vorzugsweise in o- und p-Stellung der beiden Benzolringe befinden. Demnach liegen auch die entsprechenden sulfonierten Kondensationsprodukte in der Regel als Gemische vor, insbesondere von mono- bis trisubstituierten Diphenyl-oder Diphenyletherprodukten. Je nach den Ausgangsmaterialien und den gewählten Reaktionsbedingungen bei der Herstellung der Kondensationsprodukte ändert sich das Verhältnis der Isomeren zueinander. Sofern n gleich 1 ist, werden p-Isomere in Anteilen von z.B. 30 bis 90 % und o-Isomere in Anteilen von z.B. 70 bis 10 % erhalten. Sofern n gleich 2 ist, erhält man z.B. p,p′-, o,o′- oder o,p′-Verbindungen.

Diese sulfonierten Kondensationsprodukte und deren Herstellung sind in der DE-OS 23 53 691 beschrieben. Weitere Einzelheiten können aus dieser Offenlegungsschrift entnommen werden.

Als Beispiele für die eingesetzten Emulgatoren seien ethoxylierte Wachs- oder Fettalkohole, die gegebenenfalls ganz oder teilweise mit Fettsäuren verestert sind, Polyalkohole oder vorzugsweise alkoxylierte Polyalkohole (z.B. Glykol, Diglykol, Alkylen- oder Dialkylenglykole, Sorbitan, Sorbit, Mannit, Xylit, Pentaerythrit, Diglycerin, Glycerin und Glycerylsorbit), die ganz oder teilweise mit Fettsäuren verestert sind, gegebenenfalls mit Fettsäuren veresterte ethoxylierte Zuckerderivate (z.B. Saccharose- oder Glucosederivate), Phosphorsäureester (Mono-, Di-und Triester und deren Gemische) von gegebenenfalls ethoxylierten Wachs-oder Fettalkoholen und Fettsäuremono- oder -dialkanolamide genannt. Als Ausgangsstoffe der erfindungsgemäss verwendeten Emulgatoren kommen als Wachs- oder Fettalkohole z.B. Stearyl-, Oleyl-, Cetyl-, Lanolin-, Wollfett- oder Wollwachsalkohol und als Fettsäuren z.B. Myristin-, Palmitin-, Stearin-, Isostearin-, Oel-, Linol-, Linolen- oder Lanolinsäure in Betracht. Ferner kommen auch Naturstoffe (z.B. Zoosterine oder Phytosterine), kationische Emulgatoren und hydrotope Lösungsvermittler (z.B. Polyalkohol-Polyglykol-Ether polyethoxylierter Fettsäuren) sowie Addukte aus Fett- oder Wachsalkoholen und etwa 10 bis 30 Mol Ethylen- und gegebenenfalls Propylenoxyd in Frage.

Neben den Lichtschutzmitteln aus der Klasse der sterisch gehinderten Amine können zusätzlich UV-Absorber verwendet werden. Es können alle jene Verbindungen genannt werden, die auch als UV-Absorber bekannt sind und z.B. in Kirk-Othmer 23 , 615-627; A.F. Strobel, ADR, 50 , (1961), 583-588; 51 (1962) 99-104; R. Gächter und H. Müller, Taschenbuch der Kunststoff-Additive, Carl Hanser Verlag, München S. 101-198 (1983) und in der US-A-4 511 596 beschrieben sind.

Bevorzugt eignen sich jedoch wasserlöslich gemachte UV-Absorber, die z.B. in WO 86/03528, WO 88/00942 und in der US-A-4 770 667 beschrieben sind.

Es können z.B. folgende Verbindungen eingesetzt werden:

a) 2-Hydroxybenzophenone der Formel (VII)

(VII) 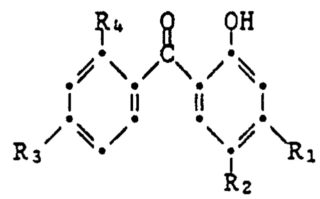 ,

worin

$R_1$ Wasserstoff, Hydroxy, $C_1$-$C_{14}$-Alkoxy oder Phenoxy,
$R_2$ Wasserstoff, Halogen, $C_1$-$C_4$-Alkyl oder Sulfo,
$R_3$ Wasserstoff, Hydroxy oder $C_1$-$C_4$-Alkoxy und
$R_4$ Wasserstoff, Hydroxy oder Carboxy
bedeuten,

wie z.B. das 4-Hydroxy, 4-Methoxy, 4-Octyloxy, 4-Decyloxy-, 4-Dodecyloxy, 4-Methoxy-2′-carboxy-, 4,2′,4′-Trihydroxy-, 4,4′-Dimethoxy-2′-hydroxy-, 4-Methoxy-5-sulfo-, 2′-Hydroxy-4,4′-dimethoxy-5-sulfo-, 4-Benzyloxy- und 5-Chlor-Derivat;

b) 2-(2′-Hydroxyphenyl)-benzotriazole der Formel (VIII)

(VIII) 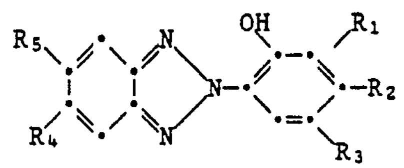 ,

worin

$R_1$ Wasserstoff, $C_1$-$C_{12}$-Alkyl, Chlor, $C_5$-$C_6$-Cycloalkyl, $C_7$-$C_9$-Phenylalkyl, oder Sulfo,
$R_2$ Wasserstoff, $C_1$-$C_4$-Alkyl, $C_1$-$C_4$-Alkoxy, Chlor, Hydroxy oder Sulfo,
$R_3$ $C_1$-$C_{12}$-Alkyl, $C_1$-$C_4$-Alkoxy, Phenyl, ($C_1$-$C_8$-Alkyl)-phenyl, $C_5$-$C_6$-Cycloalkyl, $C_2$-$C_9$-Alkoxycarbonyl, Chlor, Carboxyethyl, $C_7$-$C_9$-Phenylalkyl oder Sulfo,
$R_4$ Wasserstoff, Chlor, $C_1$-$C_4$-Alkyl, $C_1$-$C_4$-Alkoxy, $C_2$-$C_9$-Alkoxycarbonyl, Carboxy oder Sulfo und
$R_5$ Wasserstoff oder Chlor
bedeuten,

wie z.B. das 5′-Methyl-, 3′,5′-Di-tert.butyl-, 5′-tert.Butyl-, 5′-(1,1,3,3-Tetramethylbutyl)-, 5-Chlor-3′,5′-di-tert.butyl-, 5-Chlor-3′-tert.butyl-5′-methyl-, 3′-sec.Butyl-5′-tert.butyl-, 4′-Octyloxy-, 3′,5′-Di-tert.amyl- und 3′,5′-Bis-(α,α-dimethylbenzyl)Derivat.

c) 2-(2′-Hydroxyphenyl)-S-triazine der Formel (IX)

(IX) 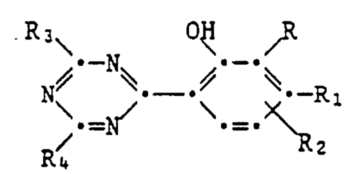

worin R Wasserstoff, Halogen, $C_1$-$C_4$-Alkyl oder Sulfo,
$R_1$ Wasserstoff, $C_1$-$C_4$-Alkyl, $C_1$-$C_4$-Alkoxy oder Hydroxy,
$R_2$ Wasserstoff oder Sulfo und
$R_3$ und $R_4$ unabhängig voneinander $C_1$-$C_4$-Alkyl, $C_1$-$C_4$-Alkoxy, $C_5$-$C_6$-Cycloalkyl, Phenyl oder durch $C_1$-$C_4$-Alkyl und Hydroxy substituiertes Phenyl bedeuten, wobei die Sulfogruppen in freier Form oder in Salzform, z.B. als Alkalimetall-, Erdalkalimetall-, Ammonium- oder Aminsalze vorliegen können. Beispiele von Verbindungen der Formel (IX) sind z.B. das 2-(2′,4′-Dihydroxyphenyl)-4,6-diphenyl-s-triazin, 2-(2′-Hydroxy-4′-methoxyphenyl)-4,6-diphenyl-s-triazin, 2-(2′-Hydroxy-4′-methoxyphenyl)-4,6-diphenyl-s-triazin, 2-(2′-Hydroxy-5′-methylphenyl)-4,6-diphenyl-s-triazin, 2,4-Bis-(2′-hydroxy-3′-methylphenyl)-6-ethyl-s-triazin, 2,4-Bis-(2′-hydroxyphenyl)-6-methoxy-s-triazin, 2,4-Bis-cyclohexyl-6-(2′-hydroxy-4′-methoxyphenyl)-s-triazin, 2-(2′-Hydroxy-4′-methoxy-5′-sulfophenyl)-4,6-diphenyl-s-triazin oder 2-(2′-Hydroxy-4′-methoxy-5′-sulfophenyl)-4(3′-sulfo-4′-methylphenyl)-6-(4′-tolyl)-s-triazin.
d) s-Triazinverbindungen der Formel

(IXa) 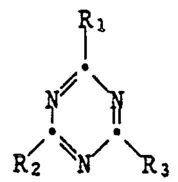 ,

worin mindestens einer der Substituenten $R_1$, $R_2$ und $R_3$ ein Rest der Formel

(IXb) 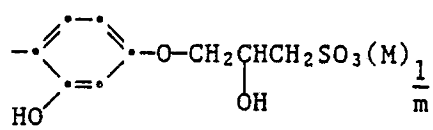

ist, worin M Natrium, Kalium, Calcium, Magnesium, Ammonium oder Tetra-$C_1$-$C_4$-alkylammonium und m 1 oder 2 ist, und der übrige Substituent bzw. die übrigen Substituenten unabhängig voneinander $C_1$-$C_{12}$-Alkyl, Phenyl, durch Sauerstoff, Schwefel, Imino oder $C_1$-$C_{11}$-Alkylimino an den Triazinylrest gebundenes $C_1$-$C_{12}$-Alkyl oder Phenyl sind, wie z.B. das Kaliumsalz der Verbindung der Formel (IXa), worin $R_2$ Phenyl und $R_2$ und $R_3$ je der Rest der Formel (IXb) bedeuten oder das Natriumsalz der Verbindung der Formel (IXa), worin $R_1$ p-Chlorphenyl und $R_2$ und $R_3$ je den Rest der Formel (IXb) bedeuten. Weitere Verbindungen sind in der EP-A-165608 beschrieben.

Die Verbindungen der Formel (VII) und (VIII) können nach an sich bekannten Verfahren, wie sie z.B. in der US-A-3 403 183 bzw. US-A-4 127 586 beschrieben sind, hergestellt werden.

Die Verbindungen der Formel (IX) können nach an sich bekannter Weise hergestellt werden, z.B. nach den in Helv. 55 , 1566-1595 (1972) beschriebenen Verfahren.

Die Applikation des dispergierten, bzw. emulgierten Lichtschutzmittels kann vor oder während dem Färben, diskontinuierlich nach einem Ausziehverfahren bei Flottenverhältnissen von 1:5 bis 1:500, vorzugsweise 1:10 bis 1:50 erfolgen. Zweckmässigerweise wird das Lichtschutzmittel direkt dem Färbebad zugesetzt.

Die Lichtschutzmittel können aber auch kontinuierlich mittels Niedrigflottenauftragssystemen oder Heissapplikationssystemen appliziert werden.

Die Menge des zugesetzten Lichtschutzmittels hängt vom Substrat und der gewünschten Stabilisierung ab. Im allgemeinen setzt man 0,01 bis 10 Gew.-%, bevorzugt 0,05 bis 5 Gew.-%, bezogen auf das Substrat, ein.

Die erfindungsgemäss anwendbaren Lichtschutzmittel können als solche oder in Form ihrer wasserlöslichen Salze eingesetzt werden. Als Salze kommen solche von organischen Säuren, wie Carbonsäuren mit 1 bis 12 C-Atomen, z.B. Ameisen-, Essig-, Propion-, Butter-, Valerian-, Capron- und Caprylsäure oder von anorganischen, mehrbasischen, Sauerstoff enthaltenden Säuren, wie z.B. Schwefelsäure oder Orthophosphonsäure in Betracht. Bevorzugt sind die Salze der Ameisen- oder Essigsäure. Salze von Verbindungen der Formel (II), worin $R_1$ Wasserstoff oder $C_1$-$C_{12}$-Alkyl bedeutet, sind von besonderem Interesse.

Bei den oligomeren Verbindungen sind solche mit einem niederen Molekulargewicht (< 700) bevorzugt.

Ein weiterer Gegenstand der vorliegenden Erfindung ist ein Mittel zur Ausführung des erfindungsgemässen Verfahrens, welches dadurch gekennzeichnet ist, dass es

1) 20 bis 80 Gew.% eines Lichtschutzmittels aus der Klasse der sterisch gehinderten Amine,
2) 0 bis 40 Gew.% eines Dispergators,
3) 0 bis 25 Gew.% eines Emulgators,
4) ad 100 Gew.% Wasser und/oder ein organisches Lösungsmittel enthält, wobei von Komponente 2 oder 3 immer nur eine eingesetzt wird.

Zweckmässigerweise enthält die Zusammensetzung des erfindungsgemässen Mittels
1) 20 bis 60 Gew.% eines Lichtschutzmittels,
2) 0 bis 30 Gew.% eines Dispergators,
3) 0 bis 15 Gew.% eines Emulgators,
4) ad 100 Gew.% Wasser und/oder ein organisches Lösungsmittel, wobei von Komponente 2 oder 3 immer nur eine eingesetzt wird.

Unter Polyamidfasermaterial wird synthetisches Polyamid, das sauer oder basisch anfärbbar ist, wie z.B. Polyamid 6, Polyamid 6,6 oder auch Polyamid 12, verstanden. Neben den reinen Polyamidfasern kommen vor allem auch Fasermischungen aus verschiedenen Polyamidfasern sowie aus Polyurethan und Polyamid in Betracht, so z.B. Trikotmaterial aus Polyamid/Polyurethan im Mischungsverhältnis 70:30. Ebenso kommen Fasermischungen aus Polypropylen und Polyamid in Frage. Grundsätzlich kann das reine oder gemischte Polyamidmaterial in verschiedenen Verarbeitungsformen vorliegen, wie z.B. als Faser, Garn, Gewebe oder Gewirke.

Das vorliegende Verfahren eignet sich besonders vorteilhaft zur Behandlung von Polyamidmaterial in Mischungen mit Polyurethan oder Polypropylen, das Licht und Hitze ausgesetzt wird und z.B. als Autopolsterstoff, Teppich oder Badebekleidungsstoff vorliegt.

Die Färbung erfolgt in üblicher Weise z.B. mit Metallkomplexfarbstoffen oder auch mit Anthrachinonfarbstoffen oder Azofarbstoffen. Als Metallkomplexfarbstoffe werden die bekannten Typen, insbesondere die 1:2-Chrom- oder 1:2-Kobaltkomplexe von Mono- oder Disazo- oder Azomethinfarbstoffen eingesetzt, die in der Literatur in grosser Zahl beschrieben sind. Neben diesen kommen natürlich auch Farbstoffe aus anderen Farbstoffklassen in Frage, wie z.B. Dispersions- oder auch Küpenfarbstoffe.

Die folgenden Herstellungsvorschriften und Beispiele dienen der Veranschaulichung der Erfindung. Teile bedeuten Gewichtsteile und Prozente Gewichtsprozente. Die Prozentangaben betreffend die Zusätze der einzelnen Behandlungs- bzw. Färbebäder beziehen sich, wenn nicht anders vermerkt, auf das Fasermaterial.

Beispiel 1:

Es werden 2 Muster à 20 g eines Polyamid-Schlingenteppichs bereitet, der zu jeweils einem Drittel aus sog. RDB -Fasern besteht (Anfärbebarkeit: R = regulär, D = tief, B = basisch).

Dieses Material wird in einem offenen Färbeapparat, z.B. einem ®AHIBA, bei einem Flottenverhältnis von 1:30 gefärbt. Dazu bereitet man 2 Flotten mit jeweils

1 % eines Alkylaminpolyglykolethers (Färbehilfsmittel)
0,5 g/l Na-acetat
0,5 ml/g Essigsäure (80 %)
} pH 5,5

Die Flotte 1 enthält bis auf die später zugesetzten Farbstoffe keine weiteren Zusätze. Der Flotte 2 wird dagegen noch 1,5 % einer Emulsion bestehend aus 75 % der Verbindung der Formel (101)

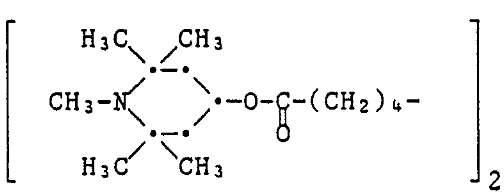

(101),

10 % eines Adduktes aus 1 Mol Nonylphenol und 9-10 Mol Ethylenoxid und 15 % eines $C_{10}$-$C_{13}$-Alkylbenzols hinzugefügt. Dann behandelt man die beiden Teppichmuster während 20 Minuten in diesen

auf 45° C erwärmten Flotten. Nach Entnahme der Muster aus den Flotten werden folgende Farbstoffe (in Wasser gelöst) jeweils in gleichen Mengen der Flotte zugesetzt:

0,1 % 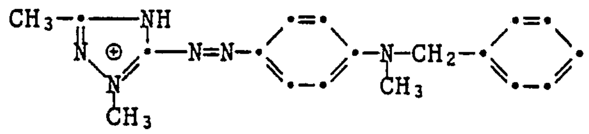 $CH_3SO_4^{\ominus}$ $Cl^{\ominus}$ $X^{\ominus}$ (102)

(C.I. Basic Red 46)

0,03 % 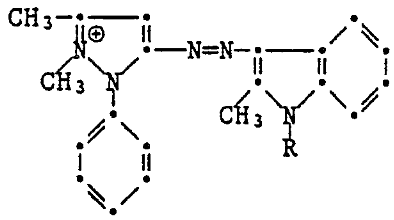 $A^{\ominus}$ R = -H, $-CH_3$ (wenig) (103)

(C.I. Basic Yellow 45)

Man gibt die beiden Muster erneut in die Flotte, behandelt noch 10 Minuten bei 40° C, erwärmt innerhalb von 30 Minuten auf 90° C und färbt bei dieser Temperatur weitere 30 Minuten. Schliesslich kühlt man auf 70° C ab, spült mit kaltem Wasser, zentrifugiert und trocknet bei 80° C.

Beide Färbungen werden auf Lichtechtheit nach SN-ISO 105-BO2 (=Xenon) und DIN 75.202 (=Fakra) geprüft.

Der Zusatz der Verbindung 101 bewirkt eine deutliche Verbesserung der Lichtechtheiten des basisch angefärbten PA und eine deutliche Hemmung der Vergilbungsneigung.

Beispiel 2:

Man verfährt wie im Beispiel 1 beschrieben mit dem Unterschied, dass die Verbindung der Formel 101 durch eine 20 %ige Dispersion der Verbindung der Formel (104), hergestellt durch Sandmahlung mit dem Na-Salz des Kondensationsprodukts aus Formaldehyd und Naphthalinsulfonat im Mengenverhältnis 1:1, ersetzt wird.

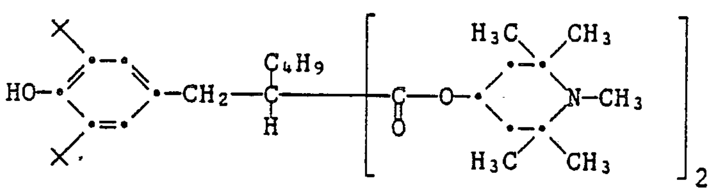 (104)

Prüft man die Lichtechtheiten dieser Färbungen, so erhält man auch mit diesem Produkt eine deutliche Verbesserung der Heisslichtechtheit nach Fakra.

Beispiel 3:

Zwei Muster eines Nylon-Teppichgarnes von 10 g werden in einem offenen Färbeapparat bei einem Flottenverhältnis von 1:30 in Flotten bei 45° C während 20 Minuten behandelt, die
0,25 % Mono-Na-phosphat und
1,75 % Di-Na-phosphat
enthalten. Die Flotte für die Färbung Nr. 2 enthält weiterhin 1,5 % der Emulsion der Verbindung der Formel (101) (siehe Beispiel 1).

Nach der Vorbehandlung erfolgt der Zusatz von 0,12 % des in Wasser gelösten Farbstoffes der Formel

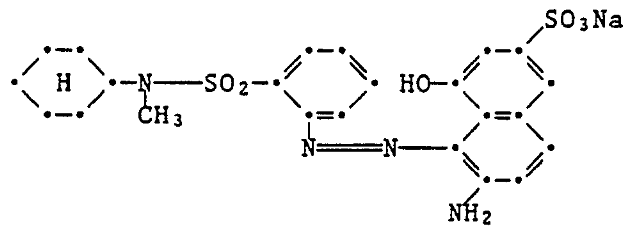

(105)

(C.I. Acid Red 361)

Beide Garnmuster werden auf ihre Lichtechtheiten [nach SN-ISO 105-BO2 (= Xenon) und DIN 75 202 (= Fakra)] geprüft. Zur Prüfung des photochemischen Abbaus werden die Garne in Einfachwicklung auf Karton gewickelt und 144 Stunden nach DIN 75.202 belichtet, sodann nach SNV 197.461 auf Reissfestigkeit und Dehnung geprüft. - In einer weiteren Prüfung werden beide Färbungen einem Hitzetest von 50 h bei 130 °C unterworfen. Die Tabelle I enthält die Testergebnisse:

Tabelle I

| FÄRBUNG | *LICHTECHTHEITEN | | **REISSFEST./DEHNUNG nach 144 h Fakra | HITZETEST |
|---|---|---|---|---|
| | XENON | FAKRA 72h | | |
| 1 | 6 | <4 | 5,3 I 15,1 % | Vergilbung und Farbstoffzerstörung bei Färbung 1 viel früher (~ 25 h) als bei Färbung 2 |
| 2 | 6 - 7 | 6 | 70,5 I 74,2 % | |

* nach Blaumassstab

** unbelichtete Färbung als Standard

Es ist ersichtlich, dass durch die Behandlung mit der Verbindung der Formel (101) Lichtechtheiten und Faserstabilität deutlich verbessert und auch die Hitzevergilbung verzögert wird.

Beispiele 4-6:

Vier Muster eines Polyamid 6-Gewirkes von 10 g Gewicht werden in einem offenen Färbeapparat bei einem Flottenverhältnis von 1:25 aus Bädern gefärbt, die jeweils

0,25 g/l Mono-Na-phosphat

1,75 g/l Di-Na-phosphat und

0,01 % des 1:2 Co-Komplexfarbstoffes der Formel (106)

(106)

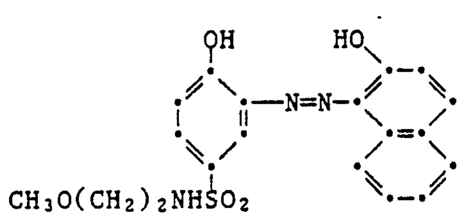

1:2 Co-Komplex

(C.I. Acid Red 251)

enthalten. Färbebad 1 % enthält keine weitere Zusätze, die Färbebäder 2-4 enthalten jeweils 1 der Verbindungen 7-9 als feingemahlene * Dispersionen

*(= Sandmahlungen mit dem Kondensationsprodukt aus Naphthalinsulfonsäure und Formaldehyd: Gew. Verhältnis 1:1)

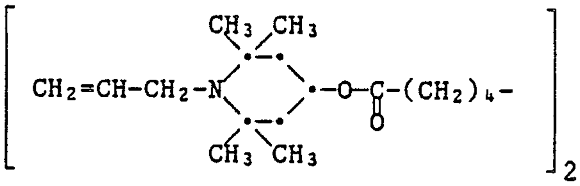

(107)

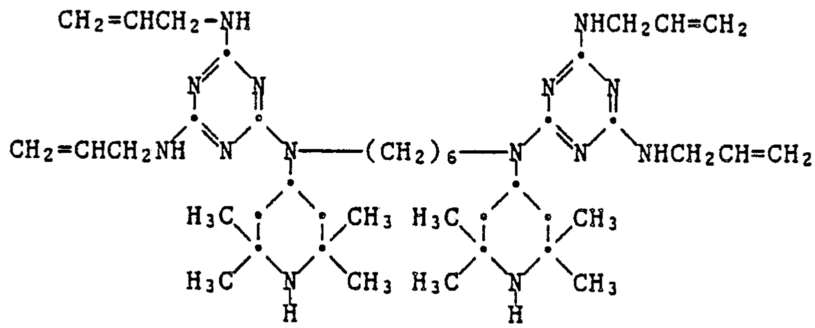

(108)

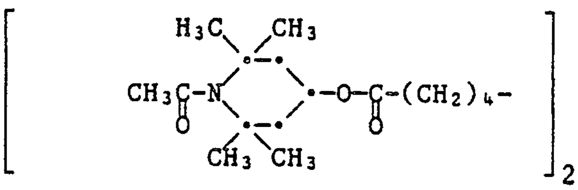

(109)

Man geht mit den Stoffmustern bei 40° C ein, erhitzt auf 95° C innerhalb von 30 Minuten und färbt bei dieser Temperatur während 30 Minuten. Die Färbungen werden gespült, zentrifugiert und getrocknet. Die Lichtechtheitsbestimmung nach DIN 75.202 (=Fakra) ergibt die in Tabelle II zusammengefassten Resultate:

Tabelle II

| FÄRBUNG | LICHTECHTHEITEN n. FAKRA* | | MECH. FESTIGKEIT nach dem BELICHTEN |
|---|---|---|---|
| | 144 h | 288 h | |
| 1 | 1 H | 1 H | nach 144/288 h : gering/keine |
| 2 | 3 H | 2 H | Muster sind noch intakt |
| 3 | -3-4 H | 2+ H | |
| 4 | -3 H | 2 H | |

* Bewertung nach Graumassstab

Beispiel 7:

Vier 10 g Muster eines ®Nylon/®Lycra-Trikots (70/30) werden in einem offenen Färbeapparat (z.B. ®AHIBA) bei einem Flottenverhältnis von 1:25 mit Flotten behandelt, die folgende Zusätze enthalten:

Flotte a: 2 % eines Alkylaminpolyglykolethers

1 % Essigsäure (80 %)

Flotte b: wie Flotte a und zusätzlich 0,75 % der Verbindung der Formel (110)

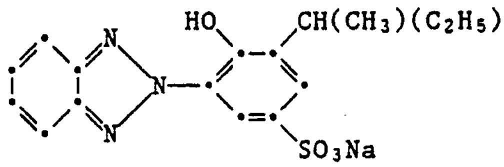

(110)

Flotte c: wie Flotte a und zusätzlich 1,5 % der Emulsion der Verbindung (101)

Flotte d: wie Flotte a und zusätzlich

0,75 % der Verbindung der Formel (110)

1,50 % der Emulsion der Verbindung (101)

Die Trikotmuster werden in den auf 50 °C erwärmten Flotten a) bis d) während 20 Minuten behandelt. Danach setzt man den Flotten a) bis d) jeweils 0,25 % des Farbstoffes der Formel (111)

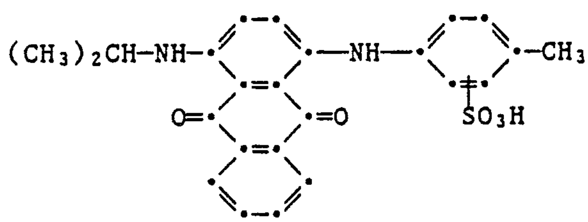

(111)

(CI. Acid Blue 258)

in gelöster Form zu. Nach einer Behandlung von 10 Minuten bei 50 °C erhitzt man das Färbebad während 30 Minuten auf 95 °C. Man färbt bei dieser Temperatur 30 Minuten und kühlt auf 70 °C ab. Danach werden die Muster kalt gespült und bei 60 °C getrocknet.

Die Lichtechtheiten der Färbungen werden nach SN-ISO 105 BO2 (Xenon) und nach DIN 75.202 (FAKRA) geprüft. Weiterhin werden Reissfestigkeit und Dehnung nach SNV 198.461 nach Belichtung von 72 h nach DIN 75.202 geprüft. Die Ergebnisse sind in Tabelle III zusammengefasst.

Tabelle III

| FÄRBUNG | LICHTECHTHEITEN | | *REISSFEST./DEHNUNG in % nach BELICHTUNG |
|---|---|---|---|
| | Xenon | Fakra | |
| Flotte a | 6 - 7 | 4 | 36/55 % |
| Flotte b | 7 | 6 + | 59/67 % |
| Flotte c | 6 - 7 | -5 | 69/76 % |
| Flotte d | 7+ | 6 - 7 | 74/87 % |

* bezogen auf Färbungen vor dem Belichten

## Ansprüche

1. Verfahren zur fotochemischen und thermischen Stabilisierung von mit sauren und basischen Farbstoffen anfärbbaren Polyamidfasern und deren Mischungen untereinander und mit anderen Fasern, dadurch gekennzeichnet, dass man das Fasermaterial mit einer wässrigen Flotte, enthaltend ein Lichtschutzmittel aus der Klasse der sterisch gehinderten Amine behandelt.

2. Verfahren gemäss Anspruch 1, worin als Lichtschutzmittel ein sterisch gehindertes Amin verwendet wird, das in seinem Molekül mindestens eine Gruppe der Formel I

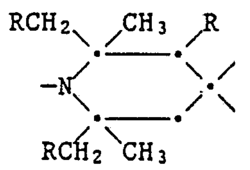

(I)

enthält, worin R Wasserstoff oder Methyl ist.

3. Verfahren gemäss einem der Ansprüche 1 oder 2, worin als Lichtschutzmittel ein sterisch gehindertes Amin der Formel II

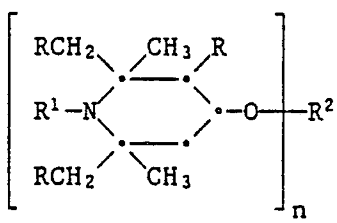

(II),

oder dessen wasserlösliche Salze verwendet werden, worin n eine Zahl von 1 bis 4, vorzugsweise 1 oder 2 bedeutet,

R Wasserstoff oder Methyl bedeutet,

$R^1$ Wasserstoff, Hydroxy, $C_1$-$C_{12}$-Alkyl, $C_3$-$C_8$-Alkenyl, $C_3$-$C_8$-Alkinyl, $C_7$-$C_{12}$-Aralkyl, $C_1$-$C_9$-Alkanoyl, $C_3$-$C_5$-Alkenoyl, Glycidyl, -O-$C_1$-$C_{12}$-Alkyl, -O-$C_1$-$C_8$-Alkanoyl oder eine Gruppe -$CH_2CH(OH)$-Z, worin Z Wasserstoff, Methyl oder Phenyl ist, bedeutet, wobei $R^1$ vorzugsweise Wasserstoff, $C_1$-$C_4$-Alkyl, Allyl, Benzyl, Acetyl oder Acryloyl ist und $R^2$, wenn n 1 ist, Wasserstoff, gegebenenfalls durch ein oder mehrere Sauerstoffatome unterbrochenes $C_1$-$C_{18}$-Alkyl, Cyanethyl, Benzyl, Glycidyl, einen einwertigen Rest einer aliphatischen, cycloaliphatischen, araliphatischen, ungesättigten oder aromatischen Carbonsäure, Carbaminsäure oder Phosphor enthaltenden Säure oder einen einwertigen Silylrest, vorzugsweise einen Rest einer aliphatischen Carbonsäure mit 2 bis 18 C-Atomen, einer cycloaliphatischen Carbonsäure mit 7 bis 15 C-Atomen, einer $\alpha,\beta$-ungesättigten Carbonsäure mit 3 bis 5 C-Atomen oder einer aromatischen Carbonsäure mit 7 bis 15 C-Atomen bedeutet, wenn n 2 ist, $C_1$-$C_{12}$-Alkylen, $C_4$-$C_{12}$-Alkenylen, Xylylen, einen zweiwertigen Rest einer aliphatischen, cycloaliphatischen, araliphatischen oder aromatischen Dicarbonsäure, Dicarbaminsäure oder Phosphor enthaltenden Säure oder einen zweiwertigen Silylrest, vorzugsweise einen Rest einer aliphatischen Dicarbonsäure mit 2 bis 36 C-Atomen, einer cycloaliphatischen oder aromatischen Dicarbonsäure mit 8 - 14 C-Atomen oder einer aliphatischen, cycloaliphatischen oder aromatischen Dicarbaminsäure mit 8 - 14 C-Atomen bedeutet, wenn n 3 ist, einen dreiwertigen Rest einer aliphatischen, cycloaliphatischen oder aromatischen Tricarbonsäure, einer aromatischen Tricarbaminsäure oder einer Phosphor enthaltenden Säure oder einen dreiwertigen Silylrest bedeutet und wenn n 4 ist, einen vierwertigen Rest einer aliphatischen, cycloaliphatischen oder aromatischen Tetracarbonsäure bedeutet.

4. Verfahren gemäss einem der Ansprüche 1 oder 2, worin als Lichtschutzmittel ein sterisch gehindertes Amin der Formel (III)

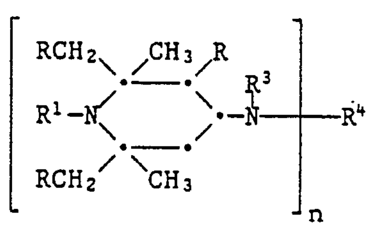

(III)

verwendet wird,

worin n die Zahl 1 oder 2 bedeutet,

R und $R^1$ die unter a) angegebene Bedeutung haben,

$R^3$ Wasserstoff, $C_1$-$C_{12}$-Alkyl, $C_2$-$C_5$-Hydroxyalkyl, $C_5$-$C_7$-Cycloalkyl, $C_7$-$C_8$-Aralkyl, $C_2$-$C_{18}$-Alkanoyl, $C_3$-$C_5$-Alkenoyl oder Benzoyl ist und

$R^4$ wenn n 1 ist, Wasserstoff, $C_1$-$C_{18}$-Alkyl, $C_3$-$C_8$-Alkenyl, $C_5$-$C_7$-Cycloalkyl, mit einer Hydroxy-, Cyano-, Alkoxycarbonyl- oder Carbamidgruppe substituiertes $C_1$-$C_4$-Alkyl, Glycidyl, eine Gruppe der Formel -$CH_2$-

CH(OH)-Z oder der Formel -CONH-Z ist, worin Z Wasserstoff, Methyl oder Phenyl bedeutet; wenn n 2 ist, $C_2$-$C_{12}$-Alkylen, $C_6$-$C_{12}$-Arylen, Xylylen, eine $-CH_2-CH(OH)-CH_2$-Gruppe oder eine Gruppe $-CH_2-CH(OH)-$CH-O-D-O- bedeutet, worin D $C_2$-$C_{10}$-Alkylen, $C_6$-$C_{15}$-Arylen, $C_6$-$C_{12}$-Cycloalkylen ist, oder vorausgesetzt, dass $R^3$ nicht Alkanoyl, Alkenoyl oder Benzoyl bedeutet, $R^4$ auch einen zweiwertigen Rest einer aliphatischen, cycloaliphatischen oder aromatischen Dicarbonsäure oder Dicarbaminsäure oder auch die Gruppe -CO- bedeuten kann, oder $R^3$ und $R^4$ zusammen, wenn n 1 ist, den zweiwertigen Rest einer aliphatischen, cycloaliphatischen oder aromatischen 1,2- oder 1,3-Dicarbonsäure bedeuten können.

5. Verfahren gemäss einem der Ansprüche 1 oder 2, dadurch gekennzeichnet, dass man als sterisch gehindertes Amin eine Verbindung der allgemeinen Formel (IV)

(IV) 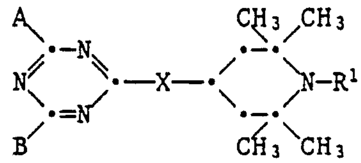

verwendet,

worin $R^1$ Wasserstoff, Oxyl-Sauerstoff, $C_1$-$C_{12}$-Alkyl, $C_3$-$C_7$-Alkenyl, $C_7$-$C_{11}$-Phenylalkyl, Cyanomethyl, $C_2$-$C_{18}$-Alkanoyl oder $C_3$-$C_{18}$-Alkenoyl, eine Gruppe $-CON(R^2)(R^3)$ oder eine Gruppe $-CH_2-CH(R^4)-OH$ bedeutet, worin

$R^2$ $C_1$-$C_{12}$-Alkyl, Allyl, Cyclohexyl, Benzyl, Phenyl oder $C_7$-$C_{12}$-Alkylphenyl und

$R^3$ Wasserstoff, $C_1$-$C_{12}$-Alkyl, Allyl oder Benzyl bedeuten oder

$R^2$ und $R^3$ zusammen mit dem N-Atom, an das sie gebunden sind, einen 5-oder 6-gliedrigen heterocyclischen Ring bilden, und

$R^4$ Wasserstoff, $C_1$-$C_{12}$-Alkyl, Phenyl, $C_2$-$C_{13}$-Alkoxymethyl oder Phenoxymethyl bedeutet,

X eine zweiwertige Gruppe der Formel -O-, $-N(R^5)-$, $-NH-(CH_2)_2-O-$, $-NH-(CH_2)_3-O-$ oder $-N(R^5)-R^7-N(R^6)-$ bedeutet, worin

$R^5$ Wasserstoff, $C_1$-$C_{12}$-Alkyl, $C_3$-$C_7$-Alkenyl, Cyclohexyl, $C_3$-$C_{12}$-Alkoxyalkyl, $C_5$-$C_{12}$-Alkenoxyalkyl, $C_4$-$C_{12}$-Dialkylaminoalkyl, eine Gruppe $-CH_2-CH(R^4)-OH$, Benzyl, eine Gruppe der Formel

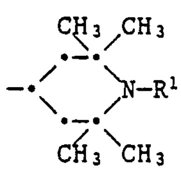

oder der Formel

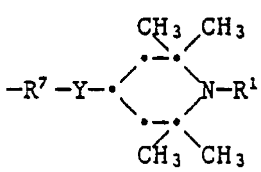

bedeutet,

$R^6$ Wasserstoff, $C_1$-$C_{12}$-Alkyl, $C_3$-$C_7$-Alkenyl, Cyclohexyl, eine Gruppe $-CH_2-CH(R^4)-OH$ oder eine Gruppe der Formel

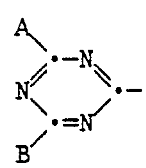

bedeutet,
$R^7$ $C_2$-$C_{12}$-Alkylen, das durch 1, 2 oder 3 der Gruppen -O- oder -N($R^6$)-unterbrochen sein kann, $C_6$-$C_{14}$-Cycloalkylen oder Cycloalkylendialkylen bedeutet,
Y eine zweiwertige Gruppe der Formel -O- oder -N($R^6$)- bedeutet und A und B, unabhängig voneinander, eine Gruppe der Formel $R^8$ O- oder ($R^9$)($R^{10}$) N- bedeuten, worin $R^8$ $C_1$-$C_{12}$-Alkyl, $C_3$-$C_7$-Alkenyl, Cyclohexyl, Benzyl, Phenyl oder $C_7$-$C_{12}$-Alkylphenyl bedeutet,
$R^9$ $C_1$-$C_{12}$-Alkyl, $C_3$-$C_7$-Alkenyl, $C_5$-$C_8$-Cycloalkyl, $C_3$-$C_{12}$-Alkoxyalkyl, $C_5$-$C_{12}$-Alkenoxyalkyl, eine Gruppe -$CH_2$-CH($R^4$)-OH, Phenyl, $C_7$-$C_{12}$-Alkylphenyl oder $C_7$-$C_{11}$-Phenylalkyl bedeutet und
$R^{10}$ Wasserstoff, $C_1$-$C_{12}$-Alkyl, $C_3$-$C_7$-Alkenyl, $C_5$-$C_8$-Cycloalkyl, $C_3$-$C_{12}$-Alkoxyalkyl, $C_5$-$C_{12}$-Alkenoxyalkyl, eine Gruppe -$CH_2$-CH($R^4$)-OH oder $C_7$-$C_{11}$-Phenylalkyl bedeutet, oder $R^9$ und $R^{10}$ zusammen mit dem N-Atom, an das sie gebunden sind, einen 5- oder 6-gliedrigen heterocyclischen Ring bilden.

6. Verfahren gemäss Anspruch 5, wobei A und B eine Gruppe der Formel $R^{15}$ O- oder ($R^{15}$)($R^{10}$)N-bedeutet, worin $R^{15}$ Allyl ist.

7. Verfahren nach einem der Ansprüche 1 bis 6, dadurch gekennzeichnet, dass man zusätzlich einen wasserlöslichen UV-Absorber verwendet.

8. Verfahren gemäss Anspruch 7, dadurch gekennzeichnet, dass man als UV-Absorber ein 2-Hydroxybenzophenon der Formel

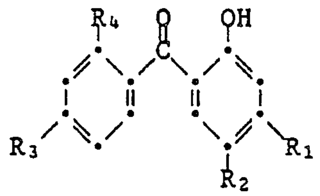

verwendet, worin
$R_1$ Wasserstoff, Hydroxy, $C_1$-$C_{14}$-Alkoxy oder Phenoxy,
$R_2$ Wasserstoff, Halogen, $C_1$-$C_4$-Alkyl oder Sulfo,
$R_3$ Wasserstoff, Hydroxy oder $C_1$-$C_4$-Alkoxy und
$R_4$ Wasserstoff, Hydroxy oder Carboxy
bedeuten,

9.Verfahren gemäss Anspruch 7, dadurch gekennzeichnet, dass man als UV-Absorber ein 2-(2'-Hydroxyphenyl)-benzotriazol der Formel

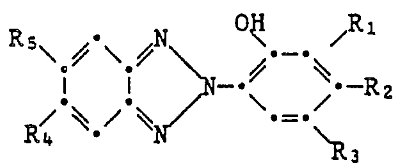

verwendet, worin
$R_1$ Wasserstoff, $C_1$-$C_{12}$-Alkyl, Chlor, $C_5$-$C_6$-Cycloalkyl, $C_7$-$C_9$-Phenylalkyl, oder Sulfo,
$R_2$ Wasserstoff, $C_1$-$C_4$-Alkyl, $C_1$-$C_4$-Alkoxy, Chlor oder Hydroxy,
$R_3$ $C_1$-$C_{12}$-Alkyl, $C_1$-$C_4$-Alkoxy, Phenyl, ($C_1$-$C_8$-Alkyl)-phenyl, $C_5$-$C_6$-Cycloalkyl, $C_2$-$C_9$-Alkoxycarbonyl, Chlor, Carboxyethyl, $C_7$-$C_9$-Phenylalkyl oder Sulfo,
$R_4$ Wasserstoff, Chlor, $C_1$-$C_4$-Alkyl, $C_1$-$C_4$-Alkoxy, $C_2$-$C_9$-Alkoxycarbonyl, Carboxy oder Sulfo und
$R_5$ Wasserstoff oder Chlor
bedeuten.

10. Verfahren gemäss Anspruch 7, dadurch gekennzeichnet, dass man als UV-Absorber ein 2-(2'-Hydroxyphenyl)-s-triazin der Formel

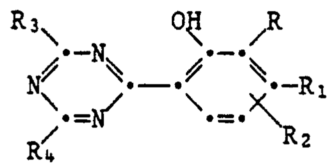

verwendet, worin R Wasserstoff, Halogen, $C_1$-$C_4$-Alkyl oder Sulfo,
$R_1$ Wassserstoff, $C_1$-$C_4$-Alkyl, $C_1$-$C_4$-Alkoxy oder Hydroxy,
$R_2$ Wasserstoff oder Sulfo bedeuten und
$R_3$ und $R_4$ unabhängig voneinander $C_1$-$C_4$-Alkyl, $C_1$-$C_4$-Alkoxy, $C_5$-$C_6$-Cycloalkyl, Phenyl oder durch $C_1$-$C_4$-Alkyl und Hydroxy substituiertes Phenyl bedeuten.

11. Verfahren gemäss Anspruch 7, dadurch gekennzeichnet, dass man als UV-Absorber eine s-Triazinverbindung der Formel

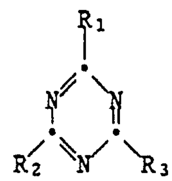

verwendet, worin mindestens einer der Substituenten $R_1$, $R_2$ und $R_3$ ein Rest der Formel

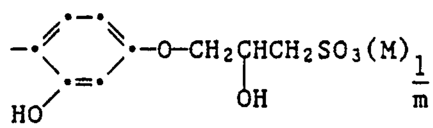

ist, worin M Natrium, Kalium, Calcium, Magnesium, Ammonium oder Tetra-$C_1$-$C_4$-alkylammonium und m 1 oder 2 ist, und der übrige Substituent bzw. die übrigen Substituenten, unabhängig voneinander $C_1$-$C_{12}$-Alkyl, Phenyl, durch Sauerstoff, Schwefel, Imino oder $C_1$-$C_{11}$-Alkylimino an den Triazinylrest gebundenes $C_1$-$C_{12}$-Alkyl oder Phenyl ist.

12. Verfahren gemäss einem der Ansprüche 1 bis 11, dadurch gekennzeichnet, dass die Polyamidfaser vor oder während dem Färben mit dem Lichtschutzmittel behandelt wird.

13. Verfahren gemäss Anspruch 12, dadurch gekennzeichnet, dass die Faser während dem Färben mit dem Lichtschutzmittel behandelt wird.

14. Verfahren gemäss einem der Ansprüche 1 bis 13, dadurch gekennzeichnet, dass die Behandlung diskontinuierlich nach einem Ausziehverfahren oder kontinuierlich vorgenommen wird.

15. Mittel zur fotochemischen und thermischen Stabilisierung von mit sauren und basischen Farbstoffen anfärbbarem Polyamidfasermaterial und deren Mischungen mit anderen Fasern, dadurch gekennzeichnet ist, dass es
1) 20 bis 80 Gew.% eines Lichtschutzmittels aus der Klasse der sterisch gehinderten Amine,
2) 0 bis 40 Gew.% eines Dispergators
3) 0 bis 25 Gew.% eines Emulgators und
4) ad 100 Gew.% Wasser und/oder ein organisches Lösungsmittel enthält,
wobei von Komponente 2 oder 3 immer nur eine eingesetzt wird.

16. Die nach einem der Ansprüche 1 bis 15 behandelten Fasermaterialien.